# EUROPÄISCHE PATENTSCHRIFT

(11) **EP 1 896 431 B1**
(45) Veröffentlichungstag und Bekanntmachung des Hinweises auf die Patenterteilung: **14.10.2009**
(21) Anmeldenummer: 06763451.9
(22) Anmeldetag: 01.06.2006
(51) Int. Cl.: C07D 251/70

(54) **VERFAHREN ZUR ENTFERNUNG VON SALZEN AUS EINEM ALKOXYCARBONYLAMINOTRIAZIN ENTHALTENDEN REAKTIONSGEMISCH**
METHOD FOR REMOVING SALTS FROM A REACTION MIXTURE CONTAINING ALKOXYCARBONYL AMINO TRIAZINE
PROCEDE D'ELIMINATION DE SELS DANS UN MELANGE REACTIONNEL CONTENANT DE L'ALCOXYCARBONYLAMINOTRIAZINE

(30) Priorität: 06.06.2005 DE 102005025900
(43) Veröffentlichungstag der Anmeldung: 12.03.2008
(73) Patentinhaber: BASF SE, 67056 Ludwigshafen (DE)
(72) Erfinder: SCHNEIDER, Jörg, 1970 Wezembeek-Oppern (DE); SCHERR, Günter, 67065 Ludwigshafen (DE); ERHARDT, Rainer, 68199 Mannheim (DE); EICHFELDER, Andreas, 67133 Maxdorf (DE); REIF, Martin, 67354 Römerberg (DE); HIRSCH, Stefan, 67435 Neustadt (DE); SIEDER, Georg, 67098 Bad-Dürkheim (DE); HOLTMANN, Thomas, 67346 Speyer (DE); CIPRIAN, Jürgen, 67071 Ludwigshafen (DE); ASCHERL, Hermann, 67246 Dirmstein (DE)
(74) Vertreter: Huhn, Michael
(86) Internationale Anmeldenummer: PCT/EP2006/062829
(87) Internationale Veröffentlichungsnummer: WO 2006/131487

(56) Entgegenhaltungen:
- WO-A-20/04054990

## Beschreibung

Die Erfindung betrifft ein Verfahren zur Entfernung von Salzen aus einem alkanolischen, bei der Herstellung von Alkoxycarbonylaminotriazinen anfallenden, mindestens ein Alkoxycarbonylaminotriazin, mindestens einen cyclischen und/oder acyclischen Kohlensäureester, mindestens ein gegebenenfalls ein oder zwei Sauerstoffatome als Etherbindung enthaltendes und gegebenenfalls durch C₁-C₄-Alkyl und/oder Hydroxy substituiertes C₁-C₁₃-Alkanol sowie mindestens ein Alkali- oder Erdalkalialkanolat, gegebenenfalls Melamin und gegebenenfalls Katalysator enthaltenden Reaktionsgemisch.

Die Herstellung von Alkoxycarbonylaminotriazinen durch Umsetzung von Triazinen, beispielsweise Melamin, mit Kohlensäureestern in Gegenwart einer Base ist zum Beispiel aus EP-A 0 624 577 bekannt. Hierbei wird in der Regel Melamin mit einem Kohlensäureester in Gegenwart des dem Kohlensäureester zugrunde liegenden Alkanols und in Gegenwart eines Alkalialkanolats, basierend auf dem dem Kohlensäureester zugrunde liegenden Alkohol, als Base zur Reaktion gebracht. Zur Aufbereitung wird dem Reaktionsgemisch eine mineralische Säure zur Neutralisation zugeführt. Als geeignete Säuren sind Phosphorsäure, Schwefelsäure und/oder Salzsäure genannt. Die Gewinnung des Alkoxycarbonylaminotriazins erfolgt anschließend durch eine Extraktion mit einem organischen Lösemittel und der Verdampfung des Lösemittels. Alternativ wird nach der Zugabe der Säure ein Feststoff durch Filtration isoliert, der dann gewaschen und getrocknet wird.

Aus der WO-A 03/035628 ist ein Verfahren zur Herstellung von Alkoxycarbonylaminotriazinen bekannt, bei welchem die Reaktionsmischung zur Aufbereitung zunächst mit einer bevorzugt wässrigen Säure neutralisiert wird. Als geeignete Säuren sind Salpetersäure, Schwefelsäure, Phosphorsäure oder deren Mischungen, aber auch Ameisensäure genannt. Nach der Zugabe der Säure zum Reaktionsgemisch bilden sich eine wässrige und eine alkanolische Phase, die voneinander getrennt werden Die alkanolische Phase enthält dabei das Alkoxycarbonylaminotriazin. Zur Erhöhung der Konzentration an Alkoxycarbonylaminotriazin wird die organische Phase nach der Abtrennung der wässrigen Phase eingeengt.

Ein entsprechendes Verfahren zur Aufbereitung eines Alkoxycarbonylaminotriazin enthaltenden Reaktionsgemisches ist auch in WO-A 2004/054990 offenbart.

Aus WO-A 2004/041922 ist ein Herstellungs- und Aufbereitungsverfahren für Carbamat-Melamin-Formaldehyd-Vernetzer bekannt. Die Aufbereitung erfolgt hierbei ebenfalls durch Zugabe einer Säure, z. B. Schwefelsäure, Ameisensäure, Oxalsäure, Phosphorsäure, Salzsäure oder Mischungen daraus. Das bei der Neutralisation entstehende Salz wird durch Filtration und Waschen mit Wasser entfernt.

Aufgabe der vorliegenden Erfindung ist es, ein Verfahren bereitzustellen, welches eine im Wesentlichen vollständige Entfernung von Salzen aus einem alkanolischen, mindestens ein Alkoxycarbonylaminotriazin enthaltenden Reaktionsgemisch ohne großen apparativen Aufwand erlaubt.

Gelöst wird die Aufgabe durch ein Verfahren zur Entfernung von Salzen aus einem alkanolischen, bei der Herstellung von Alkoxycarbonylaminotriazinen anfallenden, mindestens ein Alkoxycarbonylaminotriazin, mindestens einen cyclischen und/oder acyclischen Kohlensäureester, mindestens ein gegebenenfalls ein oder zwei Sauerstoffatome als Etherbindung enthaltendes und gegebenenfalls durch C₁-C₄-Alkyl und/oder Hydroxy substituiertes C₁-C₁₃-Alkanol sowie mindestens ein Alkali- oder Erdalkalialkanolat, gegebenenfalls Melamin und gegebenenfalls Katalysator enthaltenden Reaktionsgemisch, bei welchem Salze aus dem Reaktionsgemisch durch Ionenaustausch au einem Kationentauscher und/oder Anionentauscher entfernt werden.

Bevorzugte Alkoxycarbonylaminotriazine sind solche der allgemeinen Formel (I) 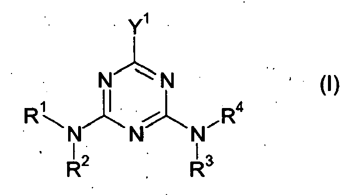 in der die Symbole und Indices die nachfolgende Bedeutung haben:

Y¹ Wasserstoff, C₁-C₄-Alkyl, gegebenenfalls durch C₁-C₄-Alkyl, C₁-C₄-Alkoxy oder Halogen substituiertes Phenyl oder ein Rest der Formel NR⁵R⁶ und

R¹, R², R³, R⁴, R⁵ und R⁶ unabhängig voneinander jeweils Wasserstoff oder ein Rest der Formel COOX oder X oder ausgewählt aus der Gruppe (-CH₂-O)ₗ,-H, (-CH₂-O)ₗ-R, (-CH₂-O)ₖ₋CH₂-N(Z)-Q und (-CH₂-O)ₖ-CH₂-N(Z)-Q, wobei
- k für 0 bis 10, vorzugsweise 1 bis 5, mehr bevorzugt 1 oder 2 und insbesondere für 1 und I für 1 bis 10, vorzugsweise 1 bis 5, mehr bevorzugt für 1 oder 2 und insbesondere 1 steht,
- R ausgewählt ist aus der Gruppe Alkyl, Cycloalkyl und Alkylaryl, wobei die Gruppen R vorzugsweise weniger als 13 Kohlenstoffatome enthalten und R bevorzugt ein C₁-C₁₃-Alkyl und besonders bevorzugt Methyl oder Butyl ist,
- Q ein Triazin-Rest der allgemeinen Formel (II) 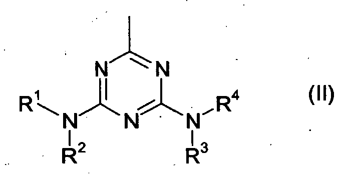 ist,
- X für C₁-C₁₃-Alkyl, dessen Kohlenstoffgerüst durch 1 oder 2 nicht benachbarte Sauerstoffatome in Etherfunktion unterbrochen und/oder durch Hydroxy substituiert sein kann oder für C₃-C₆-Alkenyl steht und
- Z für einen Rest R¹, R², R³, R⁴, R⁵ oder R⁶, wie oben definiert, steht
und
mindestens einer der Reste R¹ bis R⁴, oder wenn Y¹ für NR⁵R⁶ steht, mindestens einer der Reste R¹ bis R⁶ COOX.

Dabei bedeutet C₁-C₄-Alkyl z. B. Methyl, Ethyl, Propyl, Isopropyl, Butyl, sec-Butyl oder tert-Butyl.

Gegebenenfalls durch C₁-C₄-Alkyl, C₁-C₄-Alkoxy oder Halogen substituiertes Phenyl sind z. B. Phenyl, 2-, 3- oder 4-Methylphenyl, 2-, 3- oder 4-Ethylphenyl, 2,4-Dimethylphenyl, 2-, 3- oder 4-Methoxyphenyl, 2-, 3- oder 4-Ethoxyphenyl, 2,4-Dimethoxyphenyl, 2-, 3- oder 4-Fluorphenyl oder 2-, 3- oder 4-chlorphenyl

C₁-C₁₃-Alkyl, dessen Kohlenstoffgerüst durch 1 oder 2 nicht benachbarte Sauerstoffatome in Etherfunktion unterbrochen und/oder durch Hydroxy substituiert sein kann, bedeutet z. B. Pentyl, Isopentyl, Neopentyl, tert-Pentyl, Hexyl, 2-Methylpentyl; Heptyl, Octyl, 2-Ehtylhexyl, Isooctyl, Nonyl, Isononyl, Decyl, Isodecyl, Undecyl, Dodecyl, Tridecyl, Isotridecyl, 2-Methoxyethyl, 2-Ethoxyethyl, 2-Propoxyethyl, 2-Butoxyethyl, 2- oder 3-Methoxypropyl, 2- oder 3-Ethoxypropyl, 2- oder 3-Propoxypropyl, 2- oder 4-Methoxybutyl, 2- oder 4-Ethoxybutyl, 3,6-Dioxaheptyl, 3,6-Dioxaoctyl, 3,7-Dioxaoctyl, 4,7-Dioxaoctyl, 2- oder 3-Butoxypropyl, 2- oder 4-Butoxybutyl, 2-Hydroxyethyl, 2- oder 3-Hydroxypropyl, 2- oder 4-Hydroxybutyl, 3-Hydroxybut-2-yl. (Die obigen Bezeichnungen Isooctyl, Isononyl, Isodecyl und Isotridecyl sind Trivialbezeichnungen und stammen von den nach der Oxosynthese erhaltenen Alkoholen - vgl. Ullmanns Encyclopedia of Industrial Chemistry, 5th Edition, Vol. A1, Seiten 290 bis 293, sowie Vol. A10, Seiten 284 und 285.)

C₃-C₆-Alkenyl bedeutet z. B. Allyl, Methallyl, Ethallyl, 2-, 3- oder 4-Penten-1-yl oder 2-, 3-, 4- oder 5-Hexen-1-yl.

Gegebenenfalls ein oder zwei nicht benachbarte Sauerstoffatome als Etherbindung enthaltende und gegebenenfalls durch C₁-C₄-Alkyl und/oder Hydroxy-substituiertes C₁-C₁₃-Alkanol sind beispielsweise Methanol, Ethanol, Propanol, Isopropanol, Butanol, Isobutanol, sec-Butanol, tert-Butanol, Pentanol, Isopentanol, Neopentanol, tert-Pentanol, Hexanol, 2-Methylpentanol, Heptanol, Octanol, 2-Ethylhexanol, Isooctanol, Nonanol, Isononanol, Decanol, Isodecanol, Undecanol, Dodecanol, Tridecano, Isotridecanol, 2-Methoxyethanol, 2-Ethoxyethanol, 2-Propoxyethanol, 2-Butoxyethanol, 2-oder 3-Methoxypropanol, 2- oder 3-Ethoxypropanol, 2- oder 3-Propoxypropanol, 2-oder 4-Methoxybutanol, 2- oder 4-Ethoxybutanol, 3,6-Dioxaheptanol, 3,6-Dioxaoctanol, 3,7-Dioxaoctanol, 4,7-Dioxaoctanol, 2- oder 3-Butoxypropanol, 2- oder 4-Butoxybutanol, Ethan-1,2-diol, Propan-1,2-diol, Propan-1,3-diol, 3-Oxa-5-hydroxypentanol, 3,6-Dioxa-8-hydroxyoctanol, 3-Oxa-5-hydroxy-2,5-dimethylpentanol oder 3,6-Dioxa-8-hydroxy-2,5,8-trimethyloctanol.

Besonders bevorzugt ist das gegebenenfalls ein oder zwei nicht benachbarte Sauerstoffatome als Etherbindung enthaltende und gegebenenfalls durch C₁-C₄-Alkyl und/oder Hydroxy-substituierte C₁-C₁₃-Alkanol ausgewählt aus Methanol, Ethanol, Propanol, Isopropanol, Butanol, Isobutanol, sec-Butanol, tert-Butanol, Pentanol, Isopentanol, Neopentanol, tert-Pentanol, Hexanol, 2-Methypentanol und Heptanol.

Ganz besonders bevorzugt sind Butanol, Isobutanol, sec-Butanol und tert-Butanol sowie Mischungen aus Methanol und Butanol.

Ein cyclischer Kohlensäureester ist ein Carbonat der allgemeinen Formel (III) 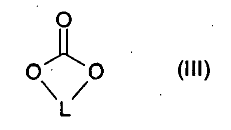 in der
L Ethylen, 1,2- oder 1,3-Propylen oder 1,2-, 1,4-, 2,3- oder 1,3-Butylen bedeutet.

Acyclische Kohlensäureester sind z. B. Diarylcarbonat, Dialkylcarbonat, Arylalkylcarbonat und Dialkenylcarbonat. Bevorzugt ist der acyclische Kohlensäureester ausgewählt aus Carbonaten der allgemeinen Formel (IV)

Z¹O-CO-OZ² (IV)

in der
Z¹ und Z² jeweils unabhängig voneinander Alkyl, Cycloalkyl und Aryl bedeuten. Bevorzugt enthalten die Reste Z¹ und Z² weniger als 13 Kohlenstoffatome. Mehr bevorzugt sind Z¹ und Z² ein C₁-C₈-Alkyl und insbesondere Methyl oder Butyl.

Bevorzugte Dialkylcarbonate sind Dimethylcarbonat, Diethylcarbonat, Dipropylcarbonat, Dibutylcarbonat und Methylbutylcarbonat.

Bevorzugte Arylalkylcarbonate sind Methylphenylcarbonat oder Butylphenylcarbonat.

Geeignete Diarylcarbonate sind z. B. Diphenylcarbonat, Di-(para-tolyl)carbonat, Di-(α-naphtyl)carbonat oder Di-(β-naphtyl)carbonat.

Ein bevorzugtes Dialkenylcarbonat ist Diallylcarbonat.

Besonders bevorzugte Kohlensäureester sind Dimethylcarbonat, Diethylcarbonat, Dibutylcarbonat, Methylbutylcarbonat, Diphenylcarbonat, Propylencarbonat oder Mischungen daraus.

Geeignete Alkali- oder Erdalkalialkanolate sind z. B. Lithium-, Natrium-, Kalium-, Magnesium- oder Calciumsalze der oben näher bezeichneten Alkanole. Die Verwendung von Alkalimethanolaten, insbesondere von Natriummethanolat, ist bevorzugt. Das Alkali- oder Erdalkalialkanolat kann entweder in festem Aggregatzustand oder in gelöster oder suspendierter Form zur Anwendung gelangen.

Bevorzugte Lösungsmittel/Verdünnungsmittel sind in diesem Fall insbesondere die oben näher bezeichneten Alkohole, allein oder als Mischung untereinander. Es können jedoch auch andere an sich bekannte und übliche inerte Verdünnungsmittel zur Anwendung gelangen.

Katalysatoren, die im Reaktionsgemisch enthalten sein können, sind Katalysatoren, die zur Herstellung des mindestens einen Alkoxycarbonylaminotriazins eingesetzt werden. Solche Katalysatoren sind z. B. Phasentransferkatalysatoren, wie sie in Ullmann's Encyclopedia of Industrial Chemistry, 5th Edition, Vol. A19, Seiten 239 bis 248 beschrieben sind. Weitere Katalysatoren können Metallsalze oder -komplexe sein, vorzugsweise Oxide, Chalkogenate, Carbonate oder Halogenide der Alkali-, Erdalkali- oder Übergangsmetalle. Zu nennen sind hier insbesondere Lithiumchlorid, Magnesiumchlorid oder Natriumcarbonat.

Im Reaktionsgemisch liegen Salze im Allgemeinen dissoziiert als Ionen vor. Erfindungsgemäß werden die Salzionen durch Ionenaustausch an einem Kationentauscher und/oder Anionentauscher aus dem Reaktionsgemisch entfernt.

Salze im Reaktionsgemisch können zum Beispiel entstehen, wenn dem alkanolischen Reaktionsgemisch zum Neutralisieren Säure zugegeben wird oder wenn das alkanolische Reaktionsgemisch in eine Säure eingetragen wird. Die Säure kann dabei konzentriert oder mit Wasser verdünnt sein. Eine gleichmäßige Verteilung der Säure im Reaktionsgemisch wird dadurch erreicht, dass während der Dosierung der Säure eine geeignete Durchmischung gewährleistet wird.

Zum Neutralisieren des Reaktionsgemisches können alle üblichen und industriell verfügbaren organischen und anorganischen Säuren in beliebiger Konzentration, vorzugsweise als 30-85 gew.-%ige wässrige Lösungen, verwendet werden. Vorzugsweise verwendet man Mineralsäuren, deren Salze eine hohe Wasserlöslichkeit aufweisen, wie Salpetersäure, Schwefelsäure oder Phosphorsäure. Eine weitere geeignete Säure ist Ameisensäure. Erfindungsgemäß ist die Verwendung von Salpetersäure besonders bevorzugt.

Erfindungsgemäß geeignete Anionentauscher sind zum Beispiel stark basische Anionentauscherharze. Bevorzugt sind vernetzte Polystyrol-Harze oder Styrol-Divinylbenzol-Copolymere mit tertiären oder quartären Aminen als funktionelle Gruppe und OH-Ionen als Austauschionen. Unter Austauschionen sind dabei die Ionen zu verstehen, die an die funktionellen Gruppen gebunden sind und gegen die aus der Flüssigkeit zu entfernenden Ionen ausgetauscht werden. Bei kommerziell erhältlichen Anionentauschern liegen die funktionellen Gruppen im Allgemeinen als Salze vor. Hierbei sind zum Beispiel Cl⁻-Ionen an die funktionelle Gruppe gebunden. Um den Anionentauscher einsetzen zu können wird dieser in diesem Fall im Allgemeinen zunächst mit NaOH vorbehandelt, um die Cl⁻-Ionen gegen OH⁻-Ionen zu tauschen. Geeignete, kommerziell erhältliche Anionentauscher sind zum Beispiel Lewatit® MP62, Lewatit® MP64 oder Lewatit® MP 600 WS der Firma Bayer AG oder auch Amberjet® 4200 CL oder Ambersep® 900 OH der Firma Rohm & Haas Co. Zur Entfernung von Nitrationen sind Ambersep® 900 OH und Lewatit® MP 600 WS bevorzugt, besonders bevorzugt ist Ambersep® 900 OH

Geeignete Kationentauscher sind zum Beispiel stark saure Kationentauscherharze auf Basis einer vernetzten Polystyrol-Matrix oder einer Styrol-Divinylbenzol-Copolymer-Matrix und Sulfonsäure als funktioneller Gruppe mit H⁺-Ionen als Austauschionen. Im Allgemeinen liegen die Kationentauscher ebenso wie die Anionentauscher in ihrer Salzform vor, wenn diese in den Handel gelangen. Um den Kationentauscher einsetzen zu können wird dieser dann im Allgemeinen mit einer Säure, z.B. Schwefelsäure, vorbehandelt, um die Kationen des Salzes durch H⁺-Ionen auszutauschen. Kommerziell erhältliche, geeignete Kationentauscher sind zum Beispiel Lewatit® S2528 oder Lewatit MonoPlus® S100 der Firma Bayer AG, Amberlyst® 40 WET und Amberjet® 1500 H der Firma Rohm & Haas Co. sowie Dowex® N306 von Dow Chemical Co. Zur Entfernung von Natriumionen werden z.B. bevorzugt Amberlyst® 40 WET und Amberjet® 1500 H eingesetzt.

In einer Ausführungsform liegen der Kationentauscher und/oder der Anionentauscher als Festbettionentauscher vor. Anstelle des Festbettionentauschers können in einer weiteren Ausführungsform der Kationentauscher und/oder der Anionentauscher auch als Granulat vorliegen. Der Kationentauscher und/oder der Anionentauscher können dabei in einem Behälter, z.B. einem Rührkessel, einer Säule, einer Kolonne oder in einem sonstigen, dem Fachmann bekannten Apparat vorliegen. Bevorzugt liegen der Kationentauscher und/oder Anionentauscher in einer Säule vor.

In einer weiteren Ausführungsform ist es auch möglich, den Kationentauscher und/oder Anionentauscher als Granulat dem Reaktor zuzuführen, in welchem das Alkoxycarbonylaminotriazin hergestellt wird. Der Reaktor ist in diesem Fall vorzugsweise ein Rührkessel.

In einer bevorzugten Ausführungsform werden die Alkalimetall- und/oder Erdalkalimetallionen mit einem Kationentauscher aus dem alkanolischen Reaktionsgemisch entfernt.

Weiterhin ist es möglich, mit einem Anionentauscher Anionen der Salze zu entfernen. So können zum Beispiel durch die Neutralisation des Reaktionsgemisches mit einer Säure Nitrat-, Sulfat- oder Phosphationen oder auch die Anionen von organischen Säuren, wie Ameisensäure, in dem Reaktionsgemisch enthalten sein, die durch den Ionenaustausch mit dem Anionentauscher entfernt werden.

Eine Regeneration des beladenen Anionentauschers erfolgt vorzugsweise mit verdünnten, mineralischen Laugen. Besonders geeignet zur Regeneration des Anionentauschers ist 5-25%ige Natronlauge.

Eine Regeneration des Kationentauschers erfolgt vorzugsweise mit verdünnten, mineralischen Säuren. Eine geeignete mineralische Säure ist z:B. 5-30%ige Salzsäure.

Zum Durchlaufen mehrerer Zyklen wird sowohl das Anionentauscherharz als auch das Kationentauscherharz im Allgemeinen mit einem Lösungsvermittler zwischen organischer und polarer Phase vorbehandelt. Hierzu wird der Ionentauscher mit einer Substanz gespült, die eine Polarität aufweist, die zwischen der Polarität der organischen und der polaren Phase liegt und vorzugsweise mit beiden Phasen mischbar ist. Zum Beispiel eignet sich Methanol als Lösungsvermittler bei Butanol als organischer Phase und Wasser als polarer Phase. Neben einer Regeneration des Ionentauscherharzes ist auch ein Verwerfen des beladenen Harzes ohne Regeneration denkbar.

Der Kationentauscher und der Anionentauscher können entweder zusammen als Gemisch, einzeln oder in hintereinander geschalteten Schritten oder Stufen eingesetzt werden. Geeignete Kombinationen geeigneter kommerziell erhältlicher Anionentauscher und Kationentauscher sind bei der Entfernung von Nitratsalzen Ambersep® 900 OH oder Amberjet® 4200 als Anionentauscher und Lewatit® S2528 als Kationentauscher. Bervorzugt ist die Kombination von Ambersep® 900 OH und Lewatit® S2528.

Der Kontakt des alkanolischen Reaktionsgemisches mit dem Kationentauscher und/oder Anionentauscher kann z.B. dadurch erfolgen, dass der Kationentauscher und/oder Anionentauscher z.B. in den Reaktor oder in einen Rührbehälter zum Reaktionsgemisch zugegeben werden, oder dadurch, dass das Reaktionsgemisch einen kontinuierlichen Ionentauscher, wobei der Ionentauscher z.B. als Packung in einem Festbett vorliegt, durchströmt.

Die Zugabe des lonentauscherharzes in den Reaktionsbehälter ist insbesondere dann möglich, wenn das mindestens eine Alkoxycarbonylaminotriazin diskontinuierlich hergestellt wird. In diesem Fall erfolgen bevorzugt sowohl die Herstellung des mindestens einen Alkoxycarbonylaminotriazins als auch gegebenenfalls eine Neutralisation des Reaktionsgemisches und die Entfernung der Salze durch Ionentausch im gleichen Behälter.

Beim Ionenaustausch werden im Allgemeinen OH-Gruppen oder Wasserstoffionen gegen die im Gemisch enthaltenen Anionen bzw. Kationen getauscht. Durch die Abgabe der OH-Gruppen oder Wasserstoffionen ändert sich der pH-Wert im Gemisch. Um einen gewünschten pH-Wert in dem das mindestens eine Alkoxycarbonylaminotriazin enthaltenden Gemisch zu erhalten, kann diesem eine Säure zur Erniedrigung des pH-Wertes oder eine Base zur Erhöhung des pH-Wertes zugegeben werden. Die Zugabe der Säure oder der Base kann dabei vor, während oder nach dem Ionenaustausch erfolgen.

Geeignete Säuren zur Einstellung des pH-Wertes sind alle mineralischen oder organischen Säuren. Um dem Effekt des Ionenaustausches nicht entgegenzuwirken werden als Säuren im Allgemeinen nicht solche eingesetzt, deren Ionen den mit dem lonentauscher entfernten Ionen entsprechen. So sollte zum Beispiel bei der Entfernung von Nitrationen aus dem Gemisch keine Salpetersäure zur Einstellung des pH-Wertes eingesetzt werden. Bevorzugt werden zur Einstellung des pH-Wertes organische Säuren eingesetzt. Besonders bevorzugt ist Ameisensäure.

Als Base zur Einstellung des pH-Wertes ist jede Base denkbar. Auch hier ist darauf zu achten, dass nicht solche Basen eingesetzt werden, deren Ionen den durch den Ionenaustausch entfernten Ionen entsprechen. Eine bevorzugte Base zur Einstellung des pH-Wertes ist Ammoniakwasser.

In einer Ausführungsform wird ein Teil der Salze vor dem Ionenaustausch durch Waschen, Extraktion oder Filtration oder Kombinationen daraus aus dem alkanolischen Reaktionsgemisch entfernt.

Das Waschen erfolgt vorzugsweise durch Zugabe von Wasser bei einer Temperatur im Bereich von 10 bis 70°C, bevorzugt von 15 bis 50°C und bei einem pH Wert von 0 bis 8, bevorzugt von 2 bis 5.

Die Extraktion wird vorzugsweise mit einem polaren, nicht vollständig mit der organischen Phase mischbaren Extraktionsmittel, durchgeführt, wobei eine alkanolische, Alkoxycarbonylaminotriazin enthaltende Phase und eine polare, Extraktionsmittel mit darin gelösten Salzen enthaltende Phase erhalten wird. Nicht vollständig mischbar bedeutet dabei, dass sich zwei Phasen mit unterschiedlicher Zusammensetzung bilden, wobei unter nicht vollständig mischbar auch verstanden wird, dass sich das Extraktionsmittel und die organische Phase überhaupt nicht mischen. Bevorzugt als Extraktionsmittel ist Wasser, besonders bevorzugt ist voll entsalztes Wasser.

Zusätzlich zu dem polaren Extraktionsmittel können bei der Extraktion Trennhilfsmittel zugegeben werden. Geeignete Trennhilfsmittel sind zum Beispiel organische Lösungsmittel oder solche, wie sie zum Beispiel in Ullmann's Encydopedia of Industrial Chemistry, Sixth Edition, 2000 Electronic Release, Abschnitt Emulsion, Kapitel 6, Breaking of Emulsions beschrieben sind.

Für die Extraktion können die dem Fachmann bekannten Apparate, z.B. Mixer/Settler-Einheiten, Kolonnen mit oder ohne Energieeintrag oder Extraktoren, die auf dem Prinzip der Zentrifugalfeldtrennung basieren, eingesetzt werden. Eine Mixer/Settler-Einheit umfasst im Allgemeinen eine Mischeinheit, wie einen Rührbehälter, eine Mischpumpe, eine Düse oder einen statischen/dynamischen Mischer. Weiterhin umfasst die Mixer/Settler-Einheit einen Abscheider, der im Allgemeinen als liegender Behälter mit oder ohne Einbauten ausgeführt ist.

Geeignete Kolonnen, die für die Extraktion eingesetzt werden können, sind z.B. Füllkörper-, Packungs- oder Siebbodenkolonnen. Geeignete Siebbodenkolonnen sind z.B. auch Querstromsiebbodenkolonnen. Als Füllkörper können alle dem Fachmann bekannten Füllkörper verwendet werden. Solche Füllkörper sind zum Beispiel in Klaus Sattler, Thermische Trennverfahren, 2. Auflage, VCH Verlagsgesellschaft mbH, Weinheim, 1995, Seiten 226 bis 229 beschrieben.

Als Packungen eignen sich geordnete oder ungeordnete Packungen. Solche Packungen sind z.B. strukturierte Packungen, Lamellenpackungen, Gewebe, Gestricke oder Gewirke.

Die Füllkörper, Packungen oder Siebböden können aus Metall oder Kunststoff gefertigt sein. Aufgrund der guten Benetzungseigenschaften von Metallen wird bei Wahl der Waschphase als kontinuierliche Phase vorzugsweise Metall als Werkstoff für die Füllkörper, Packungen oder Siebböden verwendet. Besonders geeignete Metalle sind rostfreie Edelstähle.

Neben dem Betrieb der Kolonnen mit Füllkörpern, Packungen oder Böden mit und ohne Pulsation ist auch ein Einsatz ohne Einbauten, z.B. als Sprühkolonne denkbar. Als Beispiele für geeignete, handelsübliche Extraktionskolonnen mit mechanischen Rührsystemen sind zu nennen Drehscheibenextraktoren, Old-Rushton-Kolonnen, Kühni-Extraktoren, Rührzellenextraktoren, Graesser-Extraktoren. Aber auch Zentrifugalextraktoren wiez. B. Podbielniak-Extraktoren oder Lurgi-Westfalia-Extraktoren können eingesetzt werden.

Bei der Extraktion kann die das polare, nicht vollständig mit der organischen Phase mischbare Extraktionsmittel enthaltende Waschphase entweder die kontinuierliche oder die disperse Phase der Extraktion bilden. In einer bevorzugten Form der Extraktion bildet die Waschphase die kontinuierliche Phase.

Neben der Verwendung eines einzelnen Extraktionsapparates ist es auch möglich, die Extraktion in mehreren Apparaten durchzuführen. Hierbei kann auch eine Kombination verschiedener Apparatetypen eingesetzt werden. Eine bevorzugte Kombination bilden dabei eine Mixer/Settler-Einheit und eine Füllkörperkolonne. In einer besonders bevorzugten Ausführungsform wird die Extraktion in einer Packungskolonne durchgeführt.

Bei der Extraktion liegt das Phasenverhältnis von polarer zu organischer Phase in einem Bereich von 0,1 bis 2. Bevorzugt ist ein Phasenverhältnis im Bereich von 0,15 bis 1,5, besonders bevorzugt von 0,2 bis 1 und insbesondere von 0,3 bis 0,5.

Bei Verwendung einer Packungskolonne zur Extraktion wird in einer bevorzugten Ausführungsform die polare Phase als Extrakt über den Sumpf der Kolonne abgezogen, das Raffinat - die organische Phase - läuft vorzugsweise als freier. Überlauf ab. Der Fremdphasenanteil, d.h. das Alkanol, im Extrakt wird vorzugsweise durch ein Kunststoffgestrick im Sumpf der Kolonne abgetrennt.

Bei Verwendung von Wasser als polarem Extraktionsmittel liegt der Fremdphasenanteil, d.h. der Anteil an nicht gelöstem Wasser, im Raffinat nach der Phasentrennunng im Allgemeinen bei etwa 1 Prozent.

Das Raffinat enthält das gewünschte Produkt. Sollte der Anteil an polaren und ionischen Komponenten im Raffinat größer sein als es die erforderliche Produktspezifikation erlaubt, ist es in einer bevorzugten Ausführungsform möglich, das Raffinat in den Feed zurückzuführen. Dabei kann das Raffinat z.B. entweder direkt in den Feedzulauf zur Extraktion eingespeist werden oder in einen Pufferbehälter, aus dem die Extraktion gespeist wird, zurückgeführt werden.

Die polaren und ionischen Komponenten sind z.B. Alkali- oder Erdalkalisalze, Alkali- oder Erdalkalialkanolate, Säure, cyclische oder acyclische Mono- und/oder Diester der Kohlensäure, Alkan(di)ole sowie polare Melaminderivate. Alkandiole sind z.B. Glykol und Propandiol, polare Melaminderivate sind z.B. Melamin, Mono- und Di-Alkoxycarbonylaminotriazine.

Durch die Möglichkeit, Mono- und Di-Alkoxycarbonylaminotriazine durch die Extraktion aus dem Raffinat zu entfernen, ist eine gezielte Einstellung der Verhältnisse verschiedener Alkoxycarbonylaminotriazine im Raffinat möglich.

Die Extraktion in der Packungskolonne wird im Allgemeinen als Gegenstromextraktion durchgeführt. In einer besonders bevorzugten Ausführungsform werden hierzu das polare Extraktionsmittel oberhalb der Packung und das alkanolische Reaktionsgemisch unterhalb der Packung zugeführt. Innerhalb der Kolonne strömt so das polare Extraktionsmittel durch die Packung in Richtung des Kolonnensumpfes und das alkanolische Reaktionsgemisch durch die Packung in Richtung des Kolonnenkopfes. In der Packung vermischen sich das alkanolische Reaktionsgemisch und das polare Extraktionsmittel, wobei die im alkanolischen Reaktionsgemisch enthaltenen Salze an das polare Extraktionsmittel abgegeben und so aus dem alkanolischen Reaktionsgemisch entfernt werden.

Die Temperatur, bei welcher die Extraktion durchgeführt wird, liegt vorzugsweise im Bereich von 10 bis 90°C, besonders bevorzugt im Bereich von 15 bis 50°C.

Ein bevorzugter Druck, bei dem die Extraktion durchgeführt wird, ist der Umgebungsdruck: Es ist jedoch auch möglich, die Extraktion bei einem Druck unterhalb des Umgebungsdrucks oder auch bei einem erhöhten Druck durchzuführen. Wenn die Extraktion bei erhöhtem Druck durchgeführt wird, liegt der Druck vorzugsweise im Bereich von 1 bis 10 bar.

In einer Ausführungsform kann das Verfahren als zusätzlichen Schritt das Aufkonzentrieren der organischen, mindestens ein Alkoxycarbonylaminotriazin enthaltenden Phase umfassen.

Das Aufkonzentrieren kann dabei durch thermische oder mechanische Verfahren erfolgen. Geeignete thermische Verfahren zum Aufkonzentrieren sind zum Beispiel Verdampfung, Destillation, Rektifikation, Trocknung, vorzugsweise Sprühtrocknung, oder Kristallisation. Geeignete mechanische Verfahren sind insbesondere Membrantrennverfahren, zum Beispiel Pervaporation oder Permeation sowie Filtration, wenn das mindestens eine Alkoxycarbonylaminotriazin als Suspension vorliegt. Die Verfahren zum Aufkonzentrieren können jeweils einzeln oder in Kombination zur Anwendung kommen. Es kann auch jedes weitere geeignete dem Fachmann bekannte Verfahren zur Aufkonzentrierung eingesetzt werden. Bevorzugte Verfahren zum Aufkonzentrieren sind Destillation und Sprühtrocknung.

Bei der Aufkonzentrierung durch Destillation kann diese entweder vor dem Ionenaustausch oder nach dem Ionenaustausch durchgeführt werden. In einer bevorzugten Ausführungsform erfolgt die Aufkonzentrierung durch Destillation vor dem Ionentausch. Hierbei hat es sich als vorteilhaft erwiesen, dass durch die Destillation der das mindestens eine Alkoxycarbonylaminotriazin enthaltende Volumenstrom verringert wird und so kleinere Apparate zur Durchführung des Ionentausches eingesetzt werden können. Hierdurch werden sowohl die Investitions- als auch die Betriebskosten für den Ionenaustausch gesenkt.

Das Aufkonzentrieren der organischen, mindestens ein Alkoxycarbonylaminotriazin enthaltenden Phase durch Destillation kann kontinüierlich oder diskontinuierlich erfolgen.

Zur kontinuierlichen Destillation können herkömmliche, dem Fachmann bekannte kontinuierliche Verdampfer eingesetzt werden. Geeignete Verdampfer zur kontinuierlichen Destillation sind z.B. Umlaufverdampfer, wie Robert-Selbstumlaufverdampfer, Schnellumlaufverdampfer mit schrägen Verdampferrohren, Zwangsumlaufverdampfer mit außen liegendem Verdampferbündel, Umlaufverdampfer mit in Kammern unterteiltem Siederaum oder Zwangsumlaufverdampfer mit liegendem Heizkörper. Weitere geeignete kontinuierliche Verdampfer sind z.B. Fallfilmverdampfer, Dünnschichtverdampfer oder Kestnerverdampfer.

Weiterhin kann das Aufkonzentrieren der organischen Phase durch Destillation in einer Kolonne erfolgen. Die Erwärmung auf Verdampfungstemperatur kann dabei am Kolonnensumpf erfolgen oder in einem außerhalb der Kolonne liegenden Wärmetauscher. Geeignete Kolonnen sind z.B. Packungskolonnen, Füllkörperkolonnen oder Bodenkolonnen. Geeignete Packungen, Füllkörper oder Böden sind dabei alle dem Fachmann bekannten Packungen, Füllkörper oder Böden.

Eine diskontinuierliche Aufkonzentrierung durch Destillation kann z.B. in einem Rührbehälter erfolgen. Dabei kann die Destillation auch in dem Behälter durchgeführt werden, in dem die Reaktion zu Alkoxycarbonylaminotriazin durchgeführt wird. Bevorzugt erfolgt die Aufkonzentrierung durch Destillation in einem zusätzlichen Rührbehälter.

Sowohl beim kontinuierlichen als auch beim diskontinuierlichen Verfahren fallen der das mindestens eine Alkoxycarbonylaminotriazin enthaltende Produktstrom als flüssige Phase und ein mindestens ein Alkanol, Carbonat und Wasser enthaltender Brüdenstrom an. Wenn zusätzlich zu Ionenaustausch eine Extraktion vor der Destillation durchgeführt wird, und ein von Wasser unterschiedliches polares Extraktionsmittel verwendet wird, ist im Brüden entweder anstelle des Wassers oder zusätzlich das polare Extraktionsmittel enthalten.

Die Aufkonzentrierung der organischen, mindestens ein Alkoxycarbonylaminotriazin enthaltenden Phase durch Destillation führt in einer besonders bevorzugten Ausführungsform zu einem Produktstrom, welcher 45-60 Gew.-% Alkoxycarbonylaminotriazin enthält.

Abhängig vom gewünschten Produktstrom ist es jedoch auch möglich, durch die Destillation einen Produktstrom zu erhalten, der einen kleineren oder auch einen größeren Anteil an Alkoxycarbonylaminotriazin enthält.

In einer weiteren Verfahrensvariante werden bei der Aufkonzentrierung der organischen, mindestens ein Alkoxycarbonylaminotriazin enthaltenden Phase durch Destillation die organische Phase und die polare Phase abgetrennt. Hierzu wird die organische, mindestens ein Alkoxycarbonylaminotriazin enthaltende Phase einer Destillationskolonne zugeführt. Die Destillationskolonne umfasst dabei vorzugsweise einen Verstärkungsteil und einen Abtriebsteil. Der Zulauf der organischen, mindestens ein Alkoxycarbonylaminotriazin enthaltenden Phase erfolgt vorzugsweise über einen Seitenzulauf im Verstärkungsteil.

Am Sumpf der Destillationskolonne wird die aufkonzentrierte, organische, mindestens ein Alkoxycarbonylaminotriazin enthaltende Phase gewonnen.

In einer bevorzugten Ausführungsform wird das so gewonnene Sumpfprodukt in einem Wärmetauscher zum Erwärmen der entsalzten organischen Phase, die der Destillationskolonne als Feed zugeführt wird, genutzt.

Über den Kopf der Destillationskolonne werden Alkanole, gegebenenfalls Leichtsieder und Wasser und/oder polares Extraktionsmittel abgezogen. Dieser Strom wird in einer besonders bevorzugten Ausführungsform anschließend einem Phasenscheider zugeführt, in welchem die polare Phase von der organischen Phase getrennt wird. Die organische Phase wird vorzugsweise als Rücklauf erneut der Destillationskolonne an deren Kopf zugeführt.

In einer besonders bevorzugten Ausführungsform umfasst die Destillationskolonne einen vorzugsweise im Abtriebsteil angeordneten Seitenabzug, über welchen ein vorzugsweise dampfförmiger und im Wesentlichen wasserfreier, Carbonat und Alkanol enthaltender Strom abgezogen wird. Eine besonders bevorzugte Position des Seitenabzuges ist direkt oberhalb des Kolonnensumpfes bzw. direkt unterhalb der trennwirksamen Einbauten in der Kolonne.

Vorteile dieser Betriebsweise liegen in der Rückgewinnung eines Großteils der Carbonate über Seitenabzug zum Wiedereinsatz in der Reaktion, der Reduzierung des Carbonatgehaltes im Produktstrom und in der Rückgewinnung von wasserfreien schwersiedenden Alkanolen, z.B. n-Butanol, über den Seitenabzug.

In einer bevorzugten Ausführungsform umfasst die Destillationskolonne 8 bis 22 theoretische Trennstufen.

Das Rücklaufverhältnis der organischen Phase am Kopf der Kolonne liegt vorzugsweise im Bereich zwischen 0,2 und 3 kg/kg.

Die Destillationskolonne wird vorzugsweise mit einem Druck im Bereich zwischen 20 und 2000 mbar am Kopf der Kolonne betrieben. Der bevorzugte Bereich, in welchem die Kolonne betrieben wird, liegt zwischen 50 und 950 mbar.

In einer weiteren Verfahrensvariante ist es möglich, zusätzlich zum Aufkonzentrieren der mindestens ein Alkoxycarbonylaminotriazin enthaltenden Phase durch Destillation oder anstelle des Aufkonzentrierens durch Destillation als weiteren Verfahrensschritt eine Sprühtrocknung vorzusehen. Durch die Sprühtrocknung wird pulverförmiges Alkoxycarbonylaminotriazin erzeugt.

Die Sprühtrocknung erfolgt dabei vorzugsweise in einem Sprühtrockner wie er dem Fachmann bekannt ist. So können für die Sprühtrocknung beispielsweise handelsübliche Sprühtrockner mit Zerstäuberscheibe, Einstoffdüse oder Zweistoffdüse eingesetzt werden. Der Betrieb kann je nach Bauart im Gleichstrom oder im Gegenstrom erfolgen. Bevorzugt wird eine Zweistoffdüse eingesetzt, bei der die flüssige, mindestens ein Alkoxycarbonylaminotriazin enthaltende Phase drucklos mit Hilfe eines Stickstoffstromes zerstäubt wird. Der Stickstoffstrom weist dabei einen Druck im Bereich von 1 bis 10 bar, bevorzugt im Bereich von 2 bis 5 bar und besonders bevorzugt im Bereich von 2,5 bis 5 bar auf. Der Stickstoff wird dabei vorzugsweise als Kreisgas eingesetzt.

Die Sprühtrocknung wird vorzugsweise bei einer Temperatur im Bereich von 50 bis 250°C, bevorzugt bei einer Temperatur von 55 bis 150°C und besonders bevorzugt bei einer Temperatur im Bereich von 60 bis 100°C und bei Umgebungsdruck oder einem Über- oder Unterdruck von bis zu +/- 0,01 MPa, bezogen auf den Umgebungsdruck, durchgeführt.

Das bei der Sprühtrocknung erzeugte, pulverförmige Produkt kann beispielsweise in einem Gewebefilter üblicher Bauart, wie Kerzenfilter, Sackfilter, Schlauchfilter oder anderen dem Fachmann bekannten Filtern oder in einem Zyklon abgetrennt werden. Als Filtermaterial für einen Gewebefilter eignet sich zum Beispiel Polytetrafluorethylen, Silikon oder Polyester. Bevorzugt ist Polyester.

Die Reinigung des als Kreisgas eingesetzten Stickstoffes erfolgt vorzugsweise in einem Wäscher. Dabei ist jeder beliebige, dem Fachmann bekannte Wäscher einsetzbar.

### Beispiele

### Beispiel 1

100 ml eines 50 gew.%-igen butanolischen, Alkoxycarbonylaminotriazin enthaltenden Reaktionsgemisches, welches mit 30%iger Salpetersäure neutralisiert und anschließend gewaschen wurde, wurde mit 5,078 g Anionenaustauscherharz Amberjet 4200 versetzt und 18 h bei einer Temperatur von 30°C geschüttelt. Hierdurch reduzierte sich der Nitrationengehalt im Reaktionsgemisch von 0,26 g/100 g Reaktionsmischung auf 0,026 g/100 g Reaktionsmischung.

### Beispiel 2

Das gleiche Reaktionsgemisch wie aus Beispiel 1 wurde mit 5,271 g Ambersep 900 OH Anionentauscherharz 18 h bei einer Temperatur von 30°C geschüttelt. Hierdurch reduzierte sich der Nitrationengehalt von 0,26 g/100 g Reaktionsmischung auf 0,003 g/100 g Reaktionsmischung.

### Beispiel 3

Hier wurde das Anionentauscherharz aus Beispiel 1 durch 5,119 g MP 600 WS Anionentauscherharz ausgetauscht. Hierdurch reduzierte sich der Nitrationengehalt von 0,26 g/100 g Reaktionsmischung auf 0,031 g/100 g Reaktionsmischung.

### Beispiel 4

Das in Beispiel 1 verwendete Anionenaustauscherharz wurde durch 5,570 g MP 62 Anionentauscherharz ersetzt. Hierdurch reduzierte sich der Nitrationengehalt von 0,26 g/100 g Reaktionsmischung auf 0,16 g/100 g Reaktionsmischung.

### Beispiel 5

Das Anionenaustauscherharz aus Beispiel 1 wurde durch 5,101 g MP 64 Anionentauscherharz ersetzt. Hierdurch reduzierte sich der Nitrationengehalt von 0,26 g/100 g Reaktionsmischung auf 0,1 g/100 g Reaktionsmischung. '

### Beispiel 6

Das Anionenaustauscherharz aus Beispiel 1 wurde durch 5,140 g MP 64 Anionentauscherharz und 5,016 g Kationentauscher Lewatit S2528 ersetzt. Hierdurch reduzierte sich der Nitrationengehalt von 0,26 g/100 g Reaktionsmischung auf 0,037 g/100 g Reaktionsmischung und der Natriumionengehalt von 830 mg/kg Reaktionsmischung auf 195 mg/kg Reaktionsmischung.

### Beispiel 7

Die Ionentauscher aus Beispiel 6 wurden durch 5,029 g MP 62 Anionentauscherharz und 5, 186 g Kationentauscher Lewatit S2528 ersetzt. Hierdurch reduzierte sich der Nitrationengehalt von 0,26 g/100 g Reaktionsmischung auf 0,063 g/100 g Reaktionsmischung und der Natriumionengehalt von 830 mg/kg Reaktionsmischung auf 220 mg/kg Reaktionsmischung.

### Beispiel 8

Die Ionentauscher aus Beispiel 6 wurden durch 5,004 g MP 600 WS Anionentauscherharz und 5,076 g Kationentauscher Lewatit S2528 ersetzt. Der Nitrationengehalt reduzierte sich hierdurch von 0,26 g/100 g Reaktionsmischung auf 0,016 g/100 g Reaktionsmischung und der Natriumionengehalt von 830 mg/kg Reaktionsmischung auf 150 mg/kg Reaktionsmischung.

### Beispiel 9

Die Ionentauscher aus Beispiel 6 wurden durch 5,091 g Ambersep 900 OH Anionentauscherharz und 5,089 g Kationentauscher Lewatit S2528 ersetzt. Hierdurch reduzierte sich der Nitrationengehalt von 0,26 g/100 g Reaktionsmischung auf 0,001 g/100 g Reaktionsmischung und der Natriumionengehalt von 830 mg/kg Reaktionsmischung auf 95 mg/kg Reaktionsmischung.

### Beispiel 10

Die Ionentauscher aus Beispiel 6 wurden durch 5,002 g Amberjet 4200 Anionentauscherharz und 5,005 g Kationentauscher Lewatit S2528 ersetzt. Der Nitrationengehalt reduzierte sich hierdurch von 0,26 g/100 g Reaktionsmischung auf <0,001 g/100 g Reaktionsmischung und der Natriumionengehalt von 830 mg/kg Reaktionsmischung auf 175 mg/kg Reaktionsmischung.

### Beispiel 11

100 ml eines Alkoxycarbonylaminotriazin enthaltenden, nicht neutralisierten Reaktionsgemisches wurden bei 60°C 4 Tage lang mit 50,552 g Kationentauscher Lewatit S2528 geschüttelt. Hierbei wurde eine Reduzierung des Natriumionengehalts von 30000 ppm auf 140 ppm beobachtet. Die cremeartige Reaktionsmischung wurde bei lonentauscherzugabe flüssig und es trat keine Farbänderung der Lösung auf.

### Beispiel 12

Der Kationentauscher aus Beispiel 11 wurde durch 49,363 g Kationentauscher MonoPlus S100 ersetzt. Hierbei wurde eine Reduzierung des Natriumionengehalts von 30000 ppm auf 280 ppm beobachtet. Das cremeartige Reaktionsgemisch wurde bei lonentauscherzugabe flüssig und es trat eine Farbänderung der Lösung zu einem deutlich braunen Farbton hin auf.

### Beispiel 13

Der Kationentauscher aus Beispiel 11 wurde durch 51,136 g Kationentauscher Amberlyst 40 WET ersetzt. Hierbei wurde eine Reduzierung des Natriumionengehalts von 30000 ppm auf 20 ppm beobachtet. Die cremeartige Reaktionsmischung wurde bei Ionentauscherzugabe flüssig und die Lösung wurde deutlich gelblicher

### Beispiel 14

Der Kationentauscher aus Beispiel 11 wurde durch 50,692 g Kationentauscher Amberjet 1500 H ersetzt. Hierbei wurde eine Reduzierung des Natriumionengehalts von 30000 ppm auf 10 ppm beobachtet. Die cremeartige Reaktionsmischung wurde bei lonentauscherzugabe flüssig und die Lösung wurde deutlich gelblicher.

### Beispiel 15

Der Kationentauscher aus Beispiel 11 wurde durch 50,096 g Kationentauscher Dowex N306 ersetzt. Hierbei wurde eine Reduzierung des Natriumionengehalts von 30000 ppm auf 40 ppm beobachtet. Die cremeartige Reaktionsmischung wurde bei lonentauscherzugabe flüssig und die Lösung wurde weiß.

### Beispiel 16

Zur Beurteilung des Einflusses der Vorbehandlung von Ambersep 900 OH wurden 5,036 g Ambersep 900 OH mit voll entsalztem Wasser gewaschen und 24 h bei einer Temperatur von 30°C mit 100 ml eines Alkoxycarbonylaminotriazin enthaltenden Reaktionsgemisches geschüttelt. Die Analyse erbrachte eine Reduzierung des Nitrationengehaltes von 0,26 g/100 g Reaktionsmischung auf 0,002 g/100 g Reaktionsmischung.

### Beispiel 17

5,068 g Ambersep 900 OH wurden mit voll entsalztem Wasser und anschließend mit Methanol gewaschen. Daraufhin wurde der Anionentauscher 24 h bei einer Temperatur von 30°C mit 100 ml einer Alkoxycarbonylaminotriazin enthaltenden Reaktionsmischung geschüttelt. Die Analyse erbrachte eine Reduzierung des Nitrationengehalts im Reaktionsgemisch von 0,26 g/100 g Reaktionsmischung auf 0,013 g/100 g Reaktionsmischung.

### Beispiel 18

5,077 g Ambersep 900 OH wurden mit voll entsalztem Wasser und anschließend mit Butanol gewaschen. Anschließend wurde der Anionentauscher 24 h bei einer Temperatur von 30°C mit 100 ml eines Alkoxycarbonylaminotriazin enthaltenden Reaktionsgemisches geschüttelt. Die Analyse erbrachte eine Reduzierung des Nitrationengehalts von 0,26 g/100 g Reaktionsmischung auf 0,005 g/100 g Reaktionsmischung.

### Beispiel 19

5,123 g Ambersep 900 OH wurden mit voll entsalztem Wasser und anschließend zunächst mit Methanol und dann mit Butanol gewaschen. Daraufhin wurde der Anionentauscher 24 h bei einer Temperatur von 30°C mit 100 ml neutralisiertem, Alkoxycarbonylaminotriazin enthaltenden Reaktionsgemisch geschüttelt. Die Analyse erbrachte eine Reduzierung des Nitrationengehalts von 0,26 g/100 g Reaktionsmischung auf 0,018 g/100 g Reaktionsmischung.

### Beispiel 20

Zur Beurteilung des Einflusses des Restwassergehaltes des Endprodukts auf die Flüssigphasenadsorption mit Ambersep 900 OH wurden 3,183 g Zeotith 3A mit 100 ml einer neutralisierten, Alkoxycarbonylaminotriazin enthaltenden Reaktionsmischung 4 h geschüttelt. Danach wurde das Reaktionsgemisch mit 5,133 g Ambersep 900 OH versetzt und weitere 24 h bei einer Temperatur von 30°C geschüttelt. Die Analyse erbrachte eine Reduzierung des Nitrationengehalts von 0,26 g/100 g Reaktionsmischung auf 0,001 g/100 g Reaktionsmischung.

### Beispiel 21

10,998 g Zeolith 3A wurden mit 100 ml einer neutralisierten, Alkoxycarbonylaminotriazin enthaltenden Reaktionsmischung 4 h lang geschüttelt. Danach wurde die Reaktionsmischung mit 5,091 g Ambersep 900 OH versetzt und weitere 24 h bei einer Temperatur von 30°C geschüttelt. Die Analyse erbrachte eine Reduzierung des Nitrationengehalts von 0,26 g/100 g Reaktionsmischung auf 0,001 g/100 g Reaktionsmischung.

### Beispiel 22

10,007 g Zeolith 3A wurden mit 100 ml neutralisierter, Alkoxycarbonylaminotriazin enthaltender Reaktionsmischung 4 h lang geschüttelt. Danach wurde die Reaktionsmischung mit 1,156 g Ambersep 900 OH versetzt und weitere 24 h bei einer Temperatur von 30°C geschüttelt. Die Analyse ergab eine Reduzierung des Nitrationengehalts von 0,26 g/100 g Reaktionsmischung auf 0,150 g/100 g Reaktionsmischung.

### Beispiel 23

Zwei in Reihe geschaltete Festbettkolonnen wurden mit 160 ml/h neutralisierter, Alkoxycarbonylaminotriazin enthaltender Reaktionsmischung bei 30°C 7,5 h lang betrieben. Die Umwälzung des Reaktionsgemisches erfolgte mittels einer Dosierpumpe. Die Beladung an Nitrationen in der Reaktionsmischung betrug 0,25 g/100 g Reaktionsmischung. Die Festbettkolonne 1 hatte einen Durchmesser von 10 mm, eine Schütthöhe von 470 mm und war mit Anionentauscherharz Ambersep 900 OH gefüllt. Die Festbettkolonne 2 hatte einen Durchmesser von 10 mm, eine Schütthöhe von 490 mm und war mit einem Molensieb Zeolith 3A gefüllt. Die Verweilzeit in den Kolonnen betrug 15 min und die Leerrohrgeschwindigkeit 2 m/h. Vor dem Betrieb der Festbettkolonnen wurde das Anionentauscherfestbett mit 20 Bettvolumen voll entsalztem Wasser und anschließend mit 16 Bettvolumen n-Butanol gespült. Der Durchbruch von Nitrationen erfolgte nach ca. 40 min. Die Beladung des lonentauscherharzes lag bei 0,36 val/I.

### Beispiel 24

Eine Festbettkolonne wurde 24 h lang mit einem Volumenstrom von 160 ml/h neutralisierter, Alkoxycarbonylaminotriazin enthaltender Reaktionsmischung betrieben. Die Reaktionsmischung wurde mittels einer Dosierpumpe umgewälzt. Der Nitrationengehalt in der Reaktionsmischung betrug 690 ppm. Die Festbettkolonne hatte einen Durchmesser von 20 mm, eine Schütthöhe von 955 mm und war mit Ambersep 900 OH gefüllt. Die Verweilzeit lag bei 2 h, die Leerrohrgeschwindigkeit bei 0,5 m/h. Vor dem Betrieb der Festbettkolonne mit der Reaktionsmischung wurde das Festbett mit 20 Bettvolumen voll entsalztem Wasser gespült und anschließend 8 h lang mit 4,25 Bettvolumen n-Butanol verdrängt. Der Nitrationengehalt in der das Festbett verlassene Reaktionsmischung lag stets unterhalb von 10 ppm. Nach Ablauf der 24 h wurde die Reaktionsmischung mit 1,6 Bettvolumen n-Butanol über 3 h verdrängt. Anschließend wurde das Festbett mit 6,8 Bettvolumen voll entsalztem Wasser 2 h lang gewaschen und anschließend mit 5,1 Bettvolumen 5%iger Natronlauge regeneriert. Als nächstes wurde die Festbettkolonne 2 h lang mit 6,8 Bettvolumen voll entsalztem Wasser laugenfrei gewaschen und danach 7 h lang mit 6 Bettvolumen n-Butanol wasserfrei gespült. Daraufhin wurde die Festbettkolonne 50 h lang mit einem Volumenstrom von 160 ml/h neutralisierter, Alkoxycarbonylaminotriazin enthaltender Reaktionsmischung mit einem Nitrationengehalt von 850 ppm betrieben. Vor dem Durchbruch lagen die Nitrationengehalte unterhalb von 10 ppm. Bis zum beginnenden Durchbruch wurde auf dem Anionentauscherharz eine Kapazität von 0,18 val/l erreicht.

## Patentansprüche

1. Verfahren zur Entfernung von Salzen aus einem alkanolischen, bei der Herstellung von Alkoxycarbonylaminotriazinen anfallenden, mindestens ein Alkoxycarbonylaminotriazin, mindestens einen cyclischen und/oder acyclischen Kohlensäureester, mindestens ein gegebenenfalls ein oder zwei Sauerstoffatome als Etherbindung enthaltendes und gegebenenfalls durch C₁-C₄-Alkyl und/oder Hydroxy substituiertes C₁-C₁₃-Alkanol sowie mindestens ein Alkali- oder Erdalkalialkanolat, gegebenenfalls Melamin und gegebenenfalls Katalysator enthaltenden Reaktionsgemisch, bei welchem Salze aus dem Reaktionsgemisch durch Ionenaustausch an einem Kationentauscher und/oder Anionentauscher entfernt werden.

2. Verfahren nach Anspruch 1, **dadurch gekennzeichnet, dass** der Kationentauscher und/oder der Anionentauscher als Festbettionentauscher vorliegen.

3. Verfahren nach Anspruch 1, **dadurch gekennzeichnet, dass** der Kationentauscher und/oder der Anionentauscher als Granulat vorliegen.

4. Verfahren nach einem der Ansprüche 1 bis 3, **dadurch gekennzeichnet, dass** der Anionentauscher ein stark basisches Anionentauscherharz auf Basis einer vernetzten Polystyrol-Matrix oder Styrol-Divinylbenzol-Copolymer-Matrix mit tertiären oder quartären Aminen als funktionelle Gruppe und OH⁻-Ionen als Austauschionen ist.

5. Verfahren nach einem der Ansprüche 1 bis 3, **dadurch gekennzeichnet, dass** der Kationentauscher ein stark saures Kationentauscherharz auf Basis einer vernetzten Polystyrol-Matrix oder einer Styrol-Divinylbenzol-Copolymer-Matrix mit Sulfonsäure als funktionelle Gruppe und H⁺-Ionen als Austauschionen ist.

6. Verfahren nach einem der Ansprüche 1 bis 5, **dadurch gekennzeichnet, dass** das Kationentauscherharz und oder Anionentauscherharz mit einem Lösungsvermittler zwischen organischer und polarer Phase vorbehandelt wird.

7. Verfahren nach einem der Ansprüche 1 bis 6, **dadurch gekennzeichnet, dass** der Kationentauscher und der Anionentauscher als Gemisch eingesetzt werden.

8. Verfahren nach einem der Ansprüche 1 bis 6, **dadurch gekennzeichnet, dass** der Kationentauscher und der Anionentauscher in hintereinander geschalteten Schritten eingesetzt werden.

9. Verfahren nach einem der Ansprüche 1 bis 8, **dadurch gekennzeichnet, dass** der pH-Wert des Reaktionsgemisches durch Zugabe von Säure oder Base vor, während oder nach dem Ionenaustausch eingestellt wird.

10. Verfahren nach Anspruch 1, **dadurch gekennzeichnet, dass** vor dem lonenaustausch ein Teil der Salze durch Waschen, Extraktion oder Filtration oder Kombinationen daraus aus dem Reaktionsgemisch entfernt werden.

11. Verfahren nach einem der Ansprüche 1 bis 10, **dadurch gekennzeichnet, dass** das Verfahren als weiteren Schritt das Aufkonzentrieren der organischen, Alkoxycarbonylaminotriazin enthaltenden Phase umfasst.

12. Verfahren nach Anspruch 11, **dadurch gekennzeichnet, dass** die Aufkonzentrierung der organischen, Alkoxycarbonylaminotriazin enthaltenden Phase vor dem Ionenaustausch durchgeführt wird.

13. Verfahren nach Anspruch 10, **dadurch gekennzeichnet, dass** das Waschen oder die Extraktion des neutralisierten Reaktionsgemisches mit Wasser durchgeführt wird.

14. Verfahren nach Anspruch 1, **dadurch gekennzeichnet, dass** das Alkali- oder Erdalkalialkanolat Natriummethanolat ist.

15. Verfahren nach Anspruch 1, **dadurch gekennzeichnet, dass** das C₁-C₁₃-Alkanol Butanol ist.

16. Verfahren nach Anspruch 1, **dadurch gekennzeichnet, dass** das mindestens eine gegebenenfalls ein oder zwei Sauerstoffatome als Etherbindung enthaltende und gegebenenfalls durch C₁-C₄-Alkyl und/oder Hydroxy substituierte C₁-C₁₃-Alkanol eine Mischung aus Methanol und Butanol ist.

17. Verfahren nach einem der Ansprüche 1 bis 16, **dadurch gekennzeichnet, dass** das alkanolische Reaktionsgemisch vor dem Ionentausch durch Zugabe einer Säure neutralisiert wird.

18. Verfahren nach Anspruch 17, **dadurch gekennzeichnet, dass** die zur Neutralisation zugegebene Säure Salpetersäure ist.

19. Verfahren nach einem der Ansprüche 1 bis 18, **dadurch gekennzeichnet, dass** das Verfahren als weiteren Schritt die Herstellung eines im Wesentlichen Alkoxycarbonylaminotriazin enthaltenden Pulvers durch Sprühtrocknung umfasst.

20. Verfahren nach Anspruch 19, **dadurch gekennzeichnet, dass** die Sprühtrocknung bei einer Temperatur im Bereich von 50 bis 250°C durchgeführt wird.

## Claims

1. A process for removing salts from an alkanolic reaction mixture which is obtained in the preparation of alkoxycarbonylaminotriazines and comprises at least one alkoxycarbonylaminotriazine, at least one cyclic and/or acyclic carbonic ester, at least one C₁-C₁₃-alkanol which optionally comprises one or two oxygen atoms in the form of ether bonds and is optionally substituted by C₁-C₄-alkyl and/or hydroxyl, and also at least one alkali metal alkoxide or alkaline earth metal alkoxide, with or without melamine and with or without catalyst, in which salts are removed from the reaction mixture by ion exchange over a cation exchanger and/or anion exchanger.

2. The process according to claim 1, wherein the cation exchanger and/or the anion exchanger are present in the form of fixed bed ion exchangers.

3. The process according to claim 1, wherein the cation exchanger and/or the anion exchanger are present in the form of granule.

4. The process according to any of claims 1 to 3, wherein the anion exchanger is a strongly basic anion exchange resin based on a crosslinked polystyrene matrix or styrene-divinylbenzene copolymer matrix with tertiary or quaternary amines as a functional group and OH⁻ ions as exchange ions.

5. The process according to any of claims 1 to 3, wherein the cation exchanger is a strongly acidic cation exchange resin based on a crosslinked polystyrene matrix or a styrene-divinylbenzene copolymer matrix with sulfonic acid as a functional group and H⁺ ions as exchange ions.

6. The process according to any of claims 1 to 5, wherein the cation exchange resin and/or anion exchange resin is pretreated with a solubilizer between organic and polar phase.

7. The process according to any of claims 1 to 6, wherein the cation exchanger and the anion exchanger are used in the form of a mixture.

8. The process according to any of claims 1 to 6, wherein the cation exchanger and the anion exchanger are used in steps connected in series.

9. The process according to any of claims 1 to 8, wherein the pH of the reaction mixture is adjusted by adding acid or base before, during or after the ion exchange.

10. The process according to claim 1, wherein, before the ion exchange, a portion of the salts is removed from the reaction mixture by washing, extraction or filtration or combinations thereof.

11. The process according to any of claims 1 to 10, which comprises, as a further step, the concentration of the organic phase comprising alkoxycarbonylaminotriazine.

12. The process according to claim 11, wherein the concentration of the organic phase comprising alkoxycarbonylaminotriazine is performed before the ion exchange.

13. The process according to claim 10, wherein the washing or the extraction of the neutralized reaction mixture is performed with water.

14. The process according to claim 1, wherein the alkali metal alkoxide or alkaline earth metal alkoxide is sodium methoxide.

15. The process according to claim 1, wherein the C₁-C₁₃-alkanol is butanol.

16. The process according to claim 1, wherein the at least one C₁-C₁₃-alkanol which optionally comprises one or two oxygen atoms as ether bonds and is optionally substituted by C₁-C₄-alkyl and/or hydroxyl is a mixture of methanol and butanol.

17. The process according to any of claims 1 to 16, wherein the alkanolic reaction mixture is neutralized before the ion exchange by addition of an acid.

18. The process according to claim 17, wherein the acid added for neutralization is nitric acid.

19. The process according to any of claims 1 to 18, wherein the process comprises, as a further step, the preparation of a powder comprising substantially alkoxycarbonylaminotriazine by spray drying.

20. The process according to claim 19, wherein the spray drying is carried out at a temperature in the range from 50 to 250°C.

## Revendications

1. Procédé d'élimination de sels d'un mélange réactionnel alcanolique, formé lors de la fabrication d'alcoxycarbonylaminotriazines, contenant au moins une alcoxycarbonylaminotriazine, au moins un ester d'acide carboxylique cyclique et/ou acyclique, au moins un alcanol en C₁-C₁₃ contenant éventuellement un ou deux atomes d'oxygène en tant que liaison éther et éventuellement substitué par un alkyle en C₁-C₄ et/ou un hydroxy, ainsi qu'au moins un alcanolate alcalin ou alcalino-terreux, éventuellement de la mélamine et éventuellement un catalyseur, selon lequel les sels sont éliminés du mélange réactionnel par échange d'ions sur un échangeur de cations et/ou un échangeur d'anions.

2. Procédé selon la revendication 1, **caractérisé en ce que** l'échangeur de cations et/ou l'échangeur d'anions se présentent sous la forme d'un échangeur d'ions à lit solide.

3. Procédé selon la revendication 1, **caractérisé en ce que** l'échangeur de cations et/ou l'échangeur d'anions se présentent sous la forme d'un granulat.

4. Procédé selon l'une quelconque des revendications 1 à 3, **caractérisé en ce que** l'échangeur d'anions est une résine échangeuse d'anions fortement basique à base d'une matrice de polystyrène réticulé ou d'une matrice d'un copolymère styrène-divinylbenzène avec des amines tertiaires ou quaternaires en tant que groupe fonctionnel et des ions OH⁻ en tant qu'ions d'échange.

5. Procédé selon l'une quelconque des revendications 1 à 3, **caractérisé en ce que** l'échangeur de cations est une résine échangeuse de cations fortement acide à base d'une matrice de polystyrène réticulé ou d'une matrice d'un copolymère styrène-divinylbenzène avec de l'acide sulfonique en tant que groupe fonctionnel et des ions H⁺ en tant qu'ions d'échange.

6. Procédé selon l'une quelconque des revendications 1 à 5, **caractérisé en ce que** la résine échangeuse de cations et/ou la résine échangeuse d'anions sont prétraitées avec un agent de solubilisation entre la phase organique et polaire.

7. Procédé selon l'une quelconque des revendications 1 à 6, **caractérisé en ce que** l'échangeur de cations et l'échangeur d'anions sont utilisés en mélange.

8. Procédé selon l'une quelconque des revendications 1 à 6, **caractérisé en ce que** l'échangeur de cations et l'échangeur d'anions sont utilisés lors d'étapes successives.

9. Procédé selon l'une quelconque des revendications 1 à 8, **caractérisé en ce que** la valeur de pH du mélange réactionnel est ajustée par ajout d'acide ou de base avant, pendant ou après l'échange d'ions.

10. Procédé selon la revendication 1, **caractérisé en ce qu'**une partie des sels est éliminée du mélange réactionnel avant l'échange d'ions par lavage, extraction ou filtration ou leurs combinaisons.

11. Procédé selon l'une quelconque des revendications 1 à 10, **caractérisé en ce que** le procédé comprend la concentration de la phase organique contenant l'alcoxycarbonylaminotriazine en tant qu'étape supplémentaire.

12. Procédé selon la revendication 11, **caractérisé en ce que** la concentration de la phase organique contenant l'alcoxycarbonylaminotriazine est réalisée avant l'échange d'ions.

13. Procédé selon la revendication 10, **caractérisé en ce que** le lavage ou l'extraction du mélange réactionnel neutralisé est réalisé avec de l'eau.

14. Procédé selon la revendication 1, **caractérisé en ce que** l'alcanolate alcalin ou alcalino-terreux est le méthanolate de sodium.

15. Procédé selon la revendication 1, **caractérisé en ce que** l'alcanol en C₁-C₁₃ est le butanol.

16. Procédé selon la revendication 1, **caractérisé en ce que** l'alcanol en C₁-C₁₃ contenant éventuellement un ou deux atomes d'oxygène en tant que liaison éther et éventuellement substitué par un alkyle en C₁-C₄ et/ou un hydroxy est un mélange de méthanol et de butanol.

17. Procédé selon l'une quelconque des revendications 1 à 16, **caractérisé en ce que** le mélange réactionnel alcanolique est neutralisé par ajout d'un acide avant l'échange d'ions.

18. Procédé selon la revendication 17, **caractérisé en ce que** l'acide ajouté pour la neutralisation est l'acide nitrique.

19. Procédé selon l'une quelconque des revendications 1 à 18, **caractérisé en ce que** le procédé comprend la fabrication d'une poudre contenant principalement de l'alcoxycarbonylaminotriazine par séchage par pulvérisation en tant qu'étape supplémentaire.

20. Procédé selon la revendication 19, **caractérisé en ce que** le séchage par pulvérisation est réalisé à une température dans la plage allant de 50 à 250 °C.
